# EUROPÄISCHE PATENTSCHRIFT

(11) **EP 1 924 552 B1**
(45) Veröffentlichungstag und Bekanntmachung des Hinweises auf die Patenterteilung: **10.10.2018**
(21) Anmeldenummer: 06792972.9
(22) Anmeldetag: 23.08.2006
(51) Int. Cl.: B01F 5/06, B01J 19/26, C07C 263/10, B01F 5/04

(54) **VERFAHREN UND VORRICHTUNG ZUR HERSTELLUNG VON ISOCYANATEN**
METHOD AND APPARATUS FOR PRODUCING ISOCYANATES
PROCÉDÉ ET DISPOSITIF POUR PRODUIRE DES ISOCYANATES

(30) Priorität: 06.09.2005 DE 102005042392
(43) Veröffentlichungstag der Anmeldung: 28.05.2008
(73) Patentinhaber: BASF SE, 67056 Ludwigshafen am Rhein (DE)
(72) Erfinder: WÖLFERT, Andreas, 74906 Bad Rappenau (DE)
(74) Vertreter: BASF IP Association
(86) Internationale Anmeldenummer: PCT/EP2006/065593
(87) Internationale Veröffentlichungsnummer: WO 2007/028715

(56) Entgegenhaltungen:
- EP-A- 1 449 826
- EP-A- 1 555 258

## Beschreibung

Die vorliegende Erfindung betrifft ein Verfahren zur Herstellung von Isocyanaten und eine dazu geeignete Vorrichtung, sowie deren Verwendung.

Zur Herstellung von Isocyanaten durch Phosgenierung der korrespondierenden Amine besteht prinzipiell die Möglichkeit einer Flüssigphasen- oder einer Gasphasenphosgenierung. Die Gasphasenphosgenierung zeichnet sich dadurch aus, daß die Reaktionsbedingungen so gewählt werden, daß zumindest die Reaktionskomponenten Diamin, Diisocyanat und Phosgen bei diesen Bedingungen gasförmig sind. Die vorliegende Erfindung betrifft ausschließlich die Gasphasenphosgenierung.

EP 1 275 639 A1 beschreibt die Gasphasenphosgenierung von (cyclo)aliphatischen Diaminen in einer Reaktionszone mit Einengungen der Wände.

EP 1 275 640 A1 beschreibt die Gasphasenphosgenierung von (cyclo)aliphatischen Di- und Triaminen in einem Mischrohr mit Reaktor, in dem die Gasströmung im Mischbereich beschleunigt wird. Offenbart wird eine Reaktorgeometrie mit einem Innen- und einem Außenrohr, mit der eine Gasphasenphosgenierung im Maßstab von lediglich ca. 211 g Hexamethylendiamin-1,6 pro Stunde möglich ist.

Nachteilig an den in diesen beiden Schriften offenbarten Reaktoren ist, daß bei einem einfachen Übertragen dieser Reaktorgeometrie auf großtechnisch sinnvolle Größen der Durchmesser des Innenrohres so erhöht werden müßte, daß eine Durchmischung der durch die beiden Rohre dosierten Ströme aufgrund der langen quer zur Strömungsrichtung stehenden Strecken in kurzen Mischzeiten nicht mehr möglich ist.

In EP 1 449 826 A1 wird eine Vorverteilung des aminhaltigen Stromes auf mindestens zwei einzelne Amineinleitungen offenbart. Diese sind jedoch hydrodynamisch voneinander entkoppelt. Verringert sich der Querschnitt eines einzelnen dieser Rohre, beispielsweise durch Bildung der Aminhydrochloride, so wird automatisch der Durchsatz durch dieses Rohr wegen des Druckverlustanstiegs kleiner und führt zu einer verringerten Durchströmung. Kleinere Durchsätze führen aber dazu, dass sich noch mehr Feststoffe an der Wandung festsetzten, so dass die Verstopfung noch schneller fortschreitet.

DE 10359627 A1 offenbart eine Gasphasenphosgenierung, in der Amin durch einen konzentrischen Ringspalt zwischen zwei Phosgenströme eingemischt wird, wobei die Flächenquerschnitte beider Phosgenströme in einem Verhältnis von 1:0,5 bis 1:4 stehen.

Auch an dieser Reaktorgeometrie ist nachteilig, daß bei einem einfachen Übertragen dieser Reaktorgeometrie auf großtechnisch sinnvolle Größen der Innendurchmesser des Mischorgans nur bis zu einer gewissen Grenze erhöht werden kann, damit dieses Flächenverhältnis eingehalten werden kann.

WO 02/02217 beschreibt verschiedene Verfahren zur Vermischung von Eduktströmen, untern anderem für eine Flüssigphasenphosgenierung.

Nachteilig daran ist, daß die dort offenbarten Verfahren für die Vermischung von Flüssigphasen bei niedrigen Eintrittsgeschwindigkeiten von lediglich ca. 10 m/s konzipiert sind, wohingegen die bei Gasphasenphosgenierungen erforderlichen wesentlich höheren Geschwindigkeiten und die Vermischung von Gasen andere fluiddynamische Anforderungen stellen als die Vermischung von Flüssigkeiten. Zudem sind die Reaktionsgeschwindigkeiten einer Phosgenierung in der Gasphase deutlich von denen in der Flüssigphase verschieden, so daß das Verfahren aus der WO 02/02217 nicht ohne weiteres auf die Gasphase übertragbar ist.

Aufgabe der vorliegenden Erfindung war es nun, eine Reaktionsführung für eine Gasphasenphosgenierung zu entwickeln, mit der eine großtechnische Durchführung möglich wird.

Die Aufgabe wird gelöst durch Verfahren zur Herstellung von Isocyanaten durch Umsetzung der korrespondierenden Amine mit Phosgen, gegebenenfalls in Gegenwart von mindestens einem Inertmedium, in der Gasphase, in dem in einem Reaktor n Aminströme mit n+1 Phosgenströmen umgesetzt werden, wobei n eine positive ganze Zahl von mindestens 1 ist, und alle Amin- und Phosgenströme jeweils über Ringspalte zur Vermischung in den Reaktor dosiert werden, wobei die Ringspalte eine konzentrische Anordnung um die Längsachse des Reaktors aufweisen.

In dieser Schrift werden die Begriffe "Ringspalt" und "Ringspaltraum" wie folgt verwendet:
Der Ringspaltraum wird als eine Verallgemeinerung eines Hohlzylinders aufgefaßt (zur Definition des Hohlzylinders siehe Bronstein, "Taschenbuch der Mathematik", 21. Auflage, S. 199): Ein Ringspaltraum ist das Volumen, das von einer äußeren und einer inneren, jeweils räumlich gekrümmten, geschlossenen Mantelfläche und den begrenzenden Stirnflächen eingeschlossen ist, wobei sich die beiden Mantelflächen nicht gegenseitig durchdringen.

Der Ringspalt wird als Verallgemeinerung einer Kreisringfläche aufgefaßt (zur Definition der Kreisringfläche siehe Bronstein, "Taschenbuch der Mathematik", 21. Auflage, S. 194): Der Ringspalt hat die Form der Stirnfläche, die den oben beschriebenen Ringspaltraum an dessen Stirnseite begrenzt.

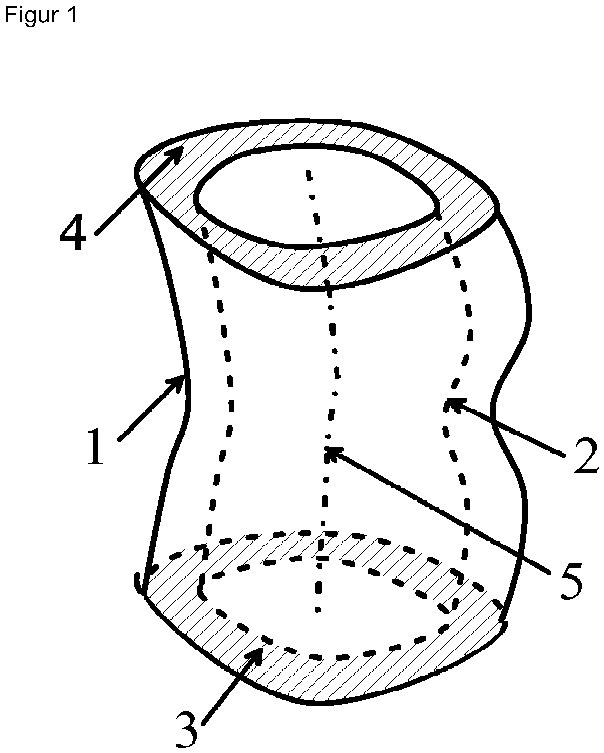

Der Ringspaltraum (siehe Figur 1) besitzt auch bei unregelmäßiger Form eine Längsachse 5. Zur Konstruktion dieser Längsachse 5 wählt man einen Punkt im Inneren des inneren Mantels 2 und sucht diejenige Ebene, die diesen Punkt enthält, und die den Ringspaltraum mit der geringsten Schnittfläche, aber vollständig in Form eines Ringspalts schneidet. Der Schwerpunkt dieser ringspaltförmigen Schnittfläche mit dem Ringspaltraum liegt auf der Längsachse des Ringspaltraums. Weitere Punkte der Längsachse 5 lassen sich durch das gleiche Vorgehen bei Wahl anderer Punkte im Inneren des inneren Mantels 2 ermitteln. Die Längsachse 5 ergibt sich als Gesamtheit aller Schwerpunkte solcher Schnittflächen. Bevorzugt bildet die Längsachse eine Gerade.

Die im Verfahren in den Ringspaltraum zu dosierenden phosgen- und aminhaltigen Eduktströme treten auf einer Seite durch (2n + 1), beispielsweise drei ringspaltförmige Flächen in den Ringspaltraum ein. Diese Ringspalte sind so zueinander angeordnet, dass ihre Schwerpunkte im wesentlichen auf einer Geraden liegen, wobei der Schwerpunkt des am weitesten in den Ringspaltraum hineinragenden Ringspalts auf der Längsachse des Ringspaltraums liegt.

Die Schwerpunkte der zur Dosierung verwendeten Ringspalte liegen in einem Punkt, so daß die Flächen konzentrisch angeordnet sind. Dann ergibt die Gesamtheit aller zur Dosierung verwendeten Ringspalte inklusive der dazwischen liegenden Stege einen Ringspalt, der eine der beiden Stirnflächen des Ringspaltraums darstellt.

"Rotationssymmetrisch" bedeutet, daß ein Körper, hier der Ringspaltraum, oder eine Fläche, hier der zugehörige Ringspalt, bei Drehung um die Rotationsachse eine Drehsymmetrie aufweist. Dabei kann es sich beispielsweise um eine zweizählige Drehachse C₂, um eine dreiachsige C₃ oder vierachsige Drehachse C₄ handeln oder bevorzugt um eine vollständige Drehsymmetrie (C_{∞}). So besitzt beispielsweise ein ebener, durch zwei Ellipsen mit jeweils zusammenfallender Längs- und Quer-Hauptachse berandeter Ringspalt eine zweizählige -Drehachse. Als weiteres Beispiel besitzt eine Kreisringfläche eine vollständige Drehsymmetrie.

Die Amine, die in eine Gasphasenphosgenierung eingesetzt werden können, müssen bestimmte Erfordernisse erfüllen (siehe unten).

Dabei kann es sich um Monoamine, Diamine, Triamine oder höherwertige Amine handeln, bevorzugt um Diamine. Ensprechend ergeben sich die korrespondierenden Monoisocyanate, Diisocyanate, Triisocyanate oder höherwertigen Isocyanate, bevorzugt Diisocyanate.

Die Amine und Isocyanate können aliphatisch, cycloaliphatisch oder aromatisch sein, bevorzugt aliphatisch oder cycloaliphatisch und besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate steht im Rahmen dieser Anmeldung kurz für cycloaliphatische und/oder aliphatische Isocyanate.

Beispiele für aromatische Diisocyanate sind sind bevorzugt solche mit 6 - 20 C-Atomen, beispielsweise monomeres 2,4'- oder 4,4'-Methylen-di(phenylisocyanat (MDI), 2,4- und/oder 2,6-Toluylendiisocyanat (TDI) und 1,5- oder 1,8-Naphtyldiisocyanat (NDI).

Diisocyanate sind bevorzugt (cyclo)aliphatische Diisocyanate, besonders bevorzugt (cyclo)aliphatische Diisocyanate mit 4 bis 20 C-Atomen.

Beispiele für übliche Diisocyanate sind aliphatische Diisocyanate wie 1,4-Tetramethylendiisocyanat, Hexamethylendiisocyanat (1,6-Diisocyanatohexan), 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanates, Tetramethylxylylendiisocyanat (TMXDI), Trimethylhexandiisocyanat oder Tetramethylhexandiisocyanat, sowie 3 (bzw. 4), 8 (bzw. 9)-Bis(isocyanatomethyl)-tricyclo[5.2.1.0^{2.6}]decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Bevorzugt sind 1,6-Diisocyanatohexan, 1-Isocyanato-3,3,5-trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanatocyclohexyl)methan.

Für das erfindungsgemäße Verfahren können solche Amine zur Reaktion zu den korrespondierenden Isocyanaten eingesetzt werden, bei denen das Amin, deren korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 mol%, besonders bevorzugt zu höchtens 1 mol% und ganz besonders bevorzugt zu höchstens 0,5 mol% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (mol/mol) Gemisch, Diaminobenzol, 2,6-Xylidin, Napthyldiamin (NDA) und 2,4'- oder 4,4'-Methylen(diphenylamin) (MDA) oder Isomerengemische davon. Bevorzugt sind unter diesen die Diamine, besonders bevorzugt ist 2,4- und/oder 2,6-TDA.

Bei der Gasphasenphosgenierung ist es definitionsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff (HCl) bilden, da die resultierenden Aminhydrochloride leicht ausfallen und nur schwer wieder verdampfbar sind.

Die Edukte, oder auch nur eines von ihnen, können zusammen mit mindestens einem Inertmedium in den Mischraum eindosiert werden.

Bei dem Inertmedium handelt es sich um ein Medium, das bei der Reaktionstemperatur gasförmig im Reaktionsraum vorliegt und nicht mit den im Reaktionsverlauf auftretenden Verbindungen reagiert. Das Inertmedium wird im allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Chlortoluol, o-Dichlorbenzol, Toluol, Xylol, Chlornaphthalin, Decahydronaphthalin, Kohlenstoffdioxid oder Kohlenstoffmonoxid, verwendet werden. Bevorzugt wird Stickstoff und/oder Chlorbenzol als Inertmedium verwendet.

Im allgemeinen wird das Inertmedium in einer Menge eingesetzt, so dass das Verhältnis der Gasvolumina von Inertmedium zu Amin bzw. zu Phosgen mehr als 0,0001 bis 30, bevorzugt mehr als 0,01 bis 15, besonders bevorzugt mehr als 0,1 bis 5 beträgt.

Die Ausgangsamine werden vor der Durchführung des erfindungsgemäßen Verfahrens verdampft und auf 200°C bis 600°C, vorzugsweise 300°C bis 500°C erhitzt und gegebenenfalls verdünnt mit einem Inertgas oder mit den Dämpfen eines inerten Lösungsmittels durch die Mischeinrichtung dem Reaktor zugeführt.

Das bei der Phosgenierung verwendete Phosgen wird vor Durchführung des erfindungsgemäßen Verfahrens gegebenenfalls verdünnt mit einem Inertgas oder mit den Dämpfen eines inerten Lösungsmittels ebenfalls auf eine Temperatur innerhalb des Bereichs von 200°C bis 600°C, vorzugsweise 300°C bis 500°C erhitzt.

In einer bevorzugten Ausführungsform werden die n Aminströme auf eine bis zu 50 °C höhere Temperatur als die (n+1) Phosgenströme erhitzt, bevorzugt auf eine bis zu 30 °C, besonders bevorzugt bis zu 24 und ganz besonders bevorzugt bis zu 20 °C höher. Bevorzugt liegt die Temperatur der n Aminströme mindestens 5 °C, besonders bevorzugt mindestens 10 °C über der der (n+1) Phosgenströme.

Erfindungsgemäß wird Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Die Vermischung und Reaktion der beiden gasförmigen Edukte findet nach dem erfindungsgemäßen Verfahren nach der Einleitung der Eduktströme Diamin und Phosgen über die Ringspalte als Eintrittsflächen in dem Ringspaltraum als Reaktionsraum statt.

Die Reaktion setzt in der Regel mit Kontakt der Edukte unmittelbar nach der Vermischung ein.

So findet im vorderen Teil des Reaktionsraums die Vermischung der Edukte, gegebenenfalls vermischt mit Inertmedium, statt (Mischraum).

Zur Durchführung der erfindungsgemäßen Umsetzung werden der vorerhitzte Strom enthaltend Amin oder Gemische von Aminen und der vorerhitzte Strom enthaltend Phosgen kontinuierlich in den Reaktor, bevorzugt einen Rohrreaktor geleitet.

Die Reaktoren bestehen im allgemeinen aus Stahl, Glas, legiertem oder emaillierten Stahl und weisen eine Länge auf, die ausreichend ist, um unter den Verfahrensbedingungen eine vollständige Umsetzung des Diamins mit dem Phosgen zu ermöglichen.

### Mischeinrichtung

Die (n+1) Phosgenströme und n Aminströme werden im allgemeinen an einem Ende des Reaktors über eine Mischeinheit einem Verweilzeitreaktor zugeführt. Zwischen jeweils 2 Phosgenströme wird über einen Ringspalt ein Aminstrom mit hoher Geschwindigkeit zugeführt. Die Zuführung aller Phosgenströme und Aminströme erfolgt jeweils über Ringspalte. Dabei handelt es sich bei den Ringspalten, mit denen benachbarte Phosgenströme und der dazwischen liegende Aminstrom zugeführt wird.

Die Zuführung erfolgt über rotationssymmetrische Ringspalte.
Die Form der Ringspalte kann beliebig sein. Denkbar sind unregelmäige Formen, trapezförmige, rechteckige, quadratische, mehreckige, ovale, ellipsenförmige oder kreisförmige Ringspalte, bevorzugt rechteckige, quadratische, ovale, ellipsenförmige oder kreisförmige und besonders bevorzugt kreisförmige Ringspalte. Durch die Form der Ringspalte wird in der Regel auch die Form der Ringspalträume bestimmt. Es kann aber auch denkbar, wenn auch weniger bevorzugt, sein, die Ringspalträume anders als die Ringspalte zu formen.

Es kann sinnvoll sein, in den Eduktzuleitungen Strömungsvergleichmäßiger einzubauen, wie sie beispielsweise aus der EP 1362847 A bekannt sind. Bevorzugt wird zur Vergleichmäßigung der Geschwindigkeit der Eduktströme jedoch eine im Verhältnis zum Durchmesser der Zuleitung lange Vorlauflänge in der Eduktzuleitung, das 2 bis 40-fache des Zuleitungsdurchmessers, besonders bevorzugt das 4 bis 30-fache, ganz besonders bevorzugt das 5 bis 20-fache.

Erfindungsgemäß entscheidend für die Durchmischung der Ströme ist eine möglichst kleine Querdiffusionsstrecke, über die die Fluidelemente sich durch turbulente und laminare Diffusion austauschen und so die Mischung bewirken. Während in der DE 103 59 627 A1 der Aminstrom über ein einen Ringspalt bildendes doppelwandiges Leitrohr dosiert wird, wird von den beiden Phosgenströmen lediglich der äußere über einen Ringspalt dosiert, nämlich über eine "Querschnittsfläche, die von der Wand des Rohrreaktors und der äußeren Wand des doppelwandigen Leitrohrs begrenzt wird", wohingegen der innere Phosgenstrom über die "Querschnittsfläche, die von der inneren Wand des doppelwandigen Leitrohrs begrenzt wird" dosiert wird, also über eine kreisförmige Fläche. Somit nimmt, der Lehre der DE 103 59 627 A1 folgend, bei einer Vergrößerung des Querschnitts des Reaktors bzw. der inneren Phosgendosierung Querdiffusionsstrecke zwischen dem inneren Phosgenstrom und dem Aminstrom zu.

Erfindungsgemäß wird der Aminstrom über einen Ringspalt zwischen zwei ihrerseits über Ringspalt dosierte Phosgenströme gemischt. Dieses Erfindungsprinzip ist analog auch für die Dosierung von n Aminströmen, beispielsweise 2, 3, 4 oder mehr, zwischen jeweils zwei Lagen von (n + 1) Phosgenströmen verallgemeinerbar.

Bevorzugt ist n 1, 2 oder 3, besonders bevorzugt 1 oder 2 und ganz besonders bevorzugt ist n = 1.

Damit der innerste Phosgenstrom dem Erfindungsgedanken folgend über einen Ringspalt dosiert werden kann, wird das Innere des Ringspalts sowie des nachfolgenden Ringspaltraums von einem Volumenkörper oder Verdränger als Kern ausgefüllt, der sich ausgehend von der Stirnfläche des Reaktionsraums in den Reaktionsraum erstreckt.

Dies bedingt erfindungsgemäß, daß es sich bei dem innersten und dem äußersten Strom jeweils um einen Phosgenstrom handelt, der den oder die Aminströme von den Wandungen des Reaktors abhält, und zwar von der Außenwand wie auch von dem inneren Volumenkörper.

Das Verhältnis der Gesamtfläche der Aminströme zur Gesamtfläche der Phosgenströme ist größer als 0,00002, bevorzugt größer als 0,0002, besonders bevorzugt größer als 0,002 und ganz besonders bevorzugt größer als 0,02.

Das Verhältnis der Gesamtfläche der Aminströme zur Gesamtfläche der Phosgenströme ist kleiner als 5, bevorzugt kleiner als 1, besonders bevorzugt kleiner als 0,5 und ganz besonders bevorzugt kleiner als 0,2.

Das Flächenverhältnis zweier phosgenführender Ringspalte, die durch einen aminführenden Ringspalt getrennt sind, beträgt 0,1 bis 10, bevorzugt 0,2 bis 5, besonders bevorzugt 0,4 bis 2,5, ganz besonders 0,8 bis 1,25, insbesondere 0,9 bis 1,1 und speziell 1.

Bei n≥2 beträgt das Flächenverhältnis zweier aminführender Ringspalte, die durch einen phosgenführenden Ringspalt getrennt sind, von 0,1 bis 10, bevorzugt 0,2 bis 5, besonders bevorzugt 0,4 bis 2,5, ganz besonders 0,8 bis 1,25, insbesondere 0,9 bis 1,1 und speziell 1.

Bevorzugt werden die einzelnen Edukte in der Mischeinrichtung mit einer Strömungsgeschwindigkeit von 20 bis 400 Meter/Sekunde in den Reaktor geführt, bevorzugt von 25 bis 300 Meter/Sekunde, besonders bevorzugt 30 bis 250, ganz besonders bevorzugt 50 bis 200, insbesondere mehr als 150 bis 200 und speziell 160 bis 180 Meter/Sekunde.

In einer möglichen Ausführungsform der Erfindung kann es sinnvoll sein, die Phosgenströme, insbesondere der äußere Phosgenstrom mit einer höheren Strömungsgeschwindigkeit durch den Ringspalt in den Mischraum einzuführen, als den Aminstrom, den sie umhüllen, besonders bevorzugt mit mindestens 10 m/s mehr, ganz besonders bevorzugt mindestens 20 m/s mehr und insbesondere mindestens 50 m/s mehr.

Es kann aber auch möglich und sinnvoll sein, den äußeren Phosgenstrom mit einer höheren Strömungsgeschwindigkeit in den Mischraum einzuführen, als den Aminstrom, und den inneren Phosgenstrom mit einer niedrigeren Strömungsgeschwindigkeit. Dies stellt eine weitere mögliche Ausführungsform der vorliegenden Erfindung dar.

In einer bevorzugten Ausführungsform der Erfindung ist es sinnvoll, die Phosgenströme, insbesondere den äußere Phosgenstrom mit einer niedrigeren Strömungsgeschwindigkeit durch die Ringspalte in den Mischraum einzuführen, als den Aminstrom, den sie umhüllen, besonders bevorzugt mit mindestens 50 m/s weniger, ganz besonders bevorzugt mindestens 120 m/s weniger, ganz besonders bevorzugt 160 m/s weniger und im speziellen mindestens 180 m/s weniger.

In einer bevorzugten Ausführungsform der vorliegenden Erfindung sind die (n+1) Ringspalte der Phosgenströme mit genau einer Phosgenzuleitung druckverlustarm ohne zusätzliche Regeleinrichtungen verbunden, so daß die Geschwindigkeit, mit der das Phosgen aus den (n+1) Ringspalten strömt, etwa gleich ist.

Desgleichen gilt, daß die n Ringspalte der Aminströme bevorzugt mit genau einer Aminzuleitung druckverlustarm ohne zusätzliche Regeleinrichtungen verbunden sind, so daß die Geschwindigkeit, mit der das Amin aus den n Ringspalten strömt, etwa gleich ist.

Es ist aber auch möglich, die Phosgen- und/oder Aminströme der Ringspalte mit jeweils einer getrennt geregelten Zuleitung zu verbinden, so daß die Geschwindigkeiten pro Zuleitung einzeln und unabhängig voneinander einstellbar sind.

Die Edukte treten mit einem Geschwindigeitsvektor in den Mischraum. Dabei lässt sich der Geschwindigkeitsvektor in eine axiale, radiale und tangentiale Richtungskomponente unterteilen. Unter der axialen Richtung wird die Richtungskomponente des Geschwindigkeitsvektors parallel zur Längsachse des Mischraums verstanden. Unter der radialen Richtung wird die Richtungskomponente des Geschwindigkeitsvektors von aussen auf die Längsachse zu verstanden, also einen rechten Winkel mit der Längsachse einschließend. Unter tangentialer Richtung wird die Richtungskomponente des Geschwindigkeitsvektors parallel zur Berandung des Ringspaltraums verstanden, also eine ringförmige Umlaufbewegung.

Zur Vermischung der Eduktströme läßt sich eine Verbesserung der sich einstellenden Vermischung durch den Einbau von eine Tangentialgeschwindigkeit erzeugenden Elementen erzielen, beispielsweise in die Zuleitung der Teilströme der Überschußkomponenten in den Mischraum. Ein geeignetes tangentialgeschwindigkeitserzeugendes Element wäre beispielsweise ein in die Zuleitung eingelassenes spiralförmig verdrilltes Band (Wendel), runde oder eckige Leitbleche (Leitschaufeln) oder dergleichen. Die Wirkung der tangentialgeschwindigkeitserzeugenden Einbauten ist es, in der Strömung der Düse die Scherung zwischen Strömungsschichten unterschiedlicher Zusammensetzung zu erhöhen.

Zur Erzeugung einer Tangentialgeschwindigkeit ist auch ein tangentialer Eintritt der Zuleitung eines oder mehrerer Eduktströme möglich oder bei einem radialen Zustrom eines oder mehrerer Eduktströme ein Schaufelkranz.

Weiterhin kann es sinnvoll sein, die Phosgen- und Aminströme mit gegenläufiger Tangentialgeschwindigkeit in den Mischraum einzuleiten, beispielsweise indem die Phosgenströme mit einer Tangentialgeschwindigkeit entlang der Längsachse des Reaktors blickend im Uhrzeigersinn, und der zwischenliegende Aminstrom mit einer Tangentialgeschwindigkeit gegen den Uhrzeigersinn in den Mischraum eindosiert werden.

Der Winkel, den der Summenvektor aus den Vektoren der Tangentialgeschwindigkeit und aus dem Vektor der Axialgeschwindigkeit der so eindosierten Ströme mit der Längsachse des Reaktors einschließt, kann von 5 bis 85°, bevorzugt 17 bis 73°, besonders bevorzugt 30 bis 60° für die einen Ströme, beispielsweise die Phosgenströme, und von -5 bis -85°, bevorzugt -17 bis --73°, besonders bevorzugt -30 bis -60° für die anderen Ströme, beispielsweise den Aminstrom betragen.

Weiterhin ist es sinnvoll, die Strömungen mit unterschiedlichen Radialgeschwindigkeiten in den Mischraum einzudosieren. Dabei stellt sich ein Winkel zwischen dem Summenvektor aus dem Radialgeschwindigkeitsvektor und aus dem Axialgeschwindigkeitsvektor mit der Längsachse ein. Dieser Winkel entspricht in der Regel dem Winkel des zugehörigen Dosierkanals mit der Längsachse des Mischraums. Dabei bedeutet eine negativer Winkel eine Dosierung von innen nach außen, ein positiver Winkel eine Dosierung von außen nach innen, ein Winkel von 0° bedeutet eine zur Längsachse des Mischraums parallele Strömung und ein Winkel von 90° eine zur Längsachse des Mischraums senkrechte Strömung.

Der äußere Phosgenstrom kann durch die Mischeinrichtung unter einem radialen Winkel von 0 bis 85°, bevorzugt 5 bis 85°, besonders bevorzugt 7 bis 65°, ganz besonders bevorzugt 15 bis 35° und insbesondere 18 bis 30° in den Mischraum eindosiert werden.

Der Aminstrom kann durch die Mischeinrichtung unter einem radialen Winkel von -50° bis +50°, bevorzugt -25 bis 25°, besonders bevorzugt -10 bis 10° und ganz besonders bevorzugt -3 bis +3° in den Mischraum eindosiert werden.

Der innere Phosgenstrom kann durch die Mischeinrichtung unter einem radialen Winkel von 0 bis -85°, bevorzugt -5 bis -85°, besonders bevorzugt -7 bis -65°, ganz besonders bevorzugt -15 bis -35° und insbesondere -18 bis -30° in den Mischraum eindosiert werden.

Vorteilhaft ist es, wenn äußerer Phosgenstrom und Aminstrom relativ zueinander einen radialen Winkel von 1 bis 45°, bevorzugt 7 bis 40, besonders bevorzugt 15 bis 35 und besonders bevorzugt 18 bis 30° einschließen.

Weiterhin vorteilhaft ist es, wenn Aminstrom und innerer Phosgenstrom relativ zueinander einen radialen Winkel von 1 bis 45°, bevorzugt 10 bis 40°, besonders bevorzugt 15 bis 35° und besonders bevorzugt 18 bis 30° einschließen.

### Reaktionsraum

Der Reaktionsraum umfaßt im vorderen Bereich den Mischraum, in dem überwiegend die Vermischung des gasförmigen Gemisches von Phosgen, Amin, gegebenenfalls vermischt mit Inertmedium, stattfindet, was in der Regel begleitet ist vom Einsetzen der Reaktion. Im hinteren Teil des Reaktionsraums findet dann im wesentlich nur noch die Reaktion statt und höchstens untergeordnet die Vermischung.

Zu Unterscheidungszwecken kann als Mischraum der Bereich des Reaktionsraumes bezeichnet werden, in dem die Vermischung der Edukte zu einem Grad von 99% stattfindet. In einer bevorzugten Ausführungsform der vorliegenden Erfindung ist der Umsatz im Mischraum, d.h. der Verbrauch des eingesetzten Amins, weniger als 15%. Dabei wird der Vermischungsgrad als Verhältnis der Differenz des lokal gemittelten Mischungsbruch und des Anfangsmischungsbruchs vor Vermischung zur Differenz des mittleren endgültigen Mischungsbruchs nach Vermischung und des Anfangsmischungsbruchs vor Vermischung angegeben. Zum Konzept des Mischungsbruches siehe z.B. J. Warnatz, U. Maas, R.W. Dibble: Verbrennung, Springer Verlag, Berlin Heidelberg New York, 1997, 2. Auflage, S. 134.

Unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht katalytischen, einphasigen Gasreaktion, geeignet sind und die den geforderten moderaten Drücken standhalten. Geeignete Materialien für den Kontakt mit dem Reaktionsgemisch sind z.B. Metalle, wie Stahl, Tantal, Silber oder Kupfer, Glas, Keramik, Emaille oder homogene oder heterogene Gemische daraus. Bevorzugt werden Stahlreaktoren verwendet. Die Wände des Reaktors können hydraulisch glatt oder profiliert sein. Als Profile eignen sich beispielsweise Ritzen oder Wellen.

Es können im allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42, auf die jeweils im Umfang dieser Offenbarung ausdrücklich Bezug genommen sei.

Anmeldungsgemäß werden Rohrreaktoren verwendet. Ebenfalls ist es möglich, im wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Technisch sinnvoll haben sich Reaktionsräume mit einem Verhältnis von Breite zu Höhe bis zu 5000 : 1, bevorzugt bis zu 1000 : 1 erwiesen.

Die Umsetzung von Phosgen mit Amin im Reaktionsraum erfolgt bei Absolutdrücken von mehr als 0,1 bar bis weniger als 20 bar, bevorzugt zwischen 0,5 bar und 15 bar und besonders bevorzugt zwischen 0,7 und 10 bar. Im Fall der Umsetzung von (cyclo)aliphatischen Aminen beträgt der Absolutdruck ganz besonders bevorzugt zwischen 0,7 bar und 5 bar, insbesondere von 0,8 bis 3 bar und speziell 1 bis 2 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum.

In einer möglichen Ausführungsform besteht der Reaktor aus einem Bündel an Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino-monocarbamoylchloride, Dicarbamoylchloride, Monoamino-monoisocyanate und Monoisocyanato-monocarbamoylchloride sowie die Hydrochloride der Aminoverbindungen.

Bei dem erfindungsgemäßen Verfahren wird die Temperatur im Reaktionsraum so gewählt, dass sie oberhalb der Siedetemperatur des eingesetzten Diamins, bezogen auf die im Reaktionsraum herrschenden Druckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600, bevorzugt bis zu 570 °C.

Die mittlere Kontaktzeit des Umsetzungsgemisches im erfindungsgemäßen Verfahren beträgt im allgemeinen zwischen 0,001 Sekunden und weniger als 5 Sekunden, bevorzugt von mehr als 0,01 Sekunden bis weniger als 3 Sekunden, besonders bevorzugt von mehr als 0,015 Sekunden bis weniger als 2 Sekunden. Im Fall der Umsetzung von (cyclo)aliphatischen Aminen kann die mittlere Kontaktzeit ganz besonders bevorzugt von 0,015 bis 1,5 Sekunden, insbesondere von 0,015 bis 0,5 Sekunden, speziell von 0,020 bis 0,1 Sekunden und oft von 0,025 bis 0,05 Sekunden betragen.

Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im Reaktor des erfindungsgemäßen Verfahrens durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktionsraums und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchläuft das gasförmige Reaktionsgemisch den Reaktionsraum mit einer Strömungsgeschwindigkeit von 10 bis 300 Meter/Sekunde, bevorzugt von 25 bis 250 Meter/Sekunde, besonders bevorzugt 40 bis 230, ganz besonders bevorzugt 50 bis 200, insbesondere mehr als 150 bis 190 und speziell 160 bis 180 Meter/Sekunde.

Durch die turbulente Strömung werden enge Verweilzeitverteilungen mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Es kann sinnvoll sein, in den Reaktor Strömungsvergleichmäßiger einzubauen, wie sie beispielsweise aus der EP 1362847 A bekannt sind.

Das Reaktionsvolumen kann über seine Außenfläche temperiert werden. Um Produktionsanlagen mit hoher Anlagenkapazität zu bauen, können mehrere Reaktorrohre parallel geschalten werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen.

In einer bevorzugten Ausführungsform werden die Umsetzungsbedingungen so gewählt, dass das Reaktionsgas am Austritt aus dem Reaktionsraum eine Phosgenkonzentration von mehr als 25 mol/m³, bevorzugt von 30 bis 50 mol/m³, aufweist. Weiterhin liegt am Austritt aus dem Reaktionsraum im allgemeinen eine Inertmediumskonzentration von mehr als 25 mol/m³, bevorzugt von 30 bis 100 mol/m³ vor.

Der Reaktionsraum kann konstruktiv im wesentlichen in bis zu vier Längenabschnitte entlang der Längsachse des Reaktors im Verlauf der Strömung zerlegt werden:
- ein erster, zumeist kurzer Abschnitt hinter der Eduktzuführeinrichtung mit der Länge L₁,
- ein zweiter Abschnitt mit der Länge L₂,
- ein dritter Abschnitt mit der Länge L₃ gefolgt von
- einem vierten Abschnitt mit der Länge L₄, der einen Rohrreaktor ohne inneren Verdrängerkörper darstellt und an den sich ein Quench (siehe unten) anschließt.

Als Reaktionsraum wird das Volumen beschrieben, in dem mindestens 98% des Umsatzes, d.h. der Verbrauch des eingesetzten Amins, stattfindet, bevorzugt mindestens 99%, besonders bevorzugt 99,5%, ganz besonders bevorzugt 99,7%, insbesondere 99,9% und speziell 99,99%.

Die einzelnen Parameter, die die Geometrien in diesen bis zu vier Abschnitten beschreiben, werden durch die Indizes 1, 2, 3 und 4 bezeichnet.

Hauptsächlich charakterisierend sind als Parameter R für den Gesamtdurchmesser des Rohrreaktors, d.h. von Innenwand zu Innenwand einschließlich des im Reaktor befindllichen Volumenkörpers, sowie r für den Außendurchmesser dieses Volumenkörpers zu nennen.

Weitere charakterisierende Parameter sind die Winkel α und β, von denen α den Winkel der Außenwand mit der Längsachse des Rohrreaktors und β den Winkel des Volumenkörpers mit der Längsachse des Rohrreaktors beschreibt.

Ein positiver Winkel α beschreibt so also eine sich im Strömungsverlauf erweiternde Außenwand des Rohrreaktors, ein negativer Winkel α dagegen eine Verjüngung.

Ein negativer Winkel β beschreibt analog einen sich in Strömungsrichtung verjüngenden Volumenkörper, ein positiver einen sich verbreiternden.

Durch die Parameter R und r wird die durchströmte Fläche F des Reaktors indirekt bestimmt, durch α und β deren Verlauf in Strömungsrichtung.

An den Übergängen von einem Abschnitt zum anderen können die Werte von R und r übereinstimmen oder aber durch Änderung zu einer sprungartigen Erweiterung/Verengung führen.

Gegenstand der vorliegenden Erfindung sind weiterhin verschiedene bevorzugte Konstruktionen des Reaktionsraums, die sich in der Größe der Querschnittsflächen entlang der Längsachse des Reaktors im Verlauf der Strömung unterscheiden:
In der ersten Konstruktionsalternative ändert sich die durchströmte Fläche F im Verlauf des Reaktors nicht, d.h. es wird keine konstruktive Unterscheidung zwischen Abschnitten getroffen, stattdessen existiert lediglich ein einziger Abschnitt 1. In dessen Verlauf bleiben r₁ und R₁ über die gesamte Länge des Reaktors gleich, α₁ und β₁ sind jeweils 0°. Der gesamte Reaktor wird also aus einem Ringspaltraum gebildet, bevorzugt aus einem rotationssymmetrischen Ringspaltraum, besonders bevorzugt aus einem Hohlzylinder.

In einer zweiten Konstruktionsalternative vergrößert sich die durchströmte Fläche F₁ in einem Abschnitt 1 und bleibt danach in einem zweiten Abschnitt 2 konstant.

Dies wird bevorzugt dadurch erreicht, daß man R₁ konstant hält und r₁ im Verlauf des Abschnittes 1 auf Null verringert. Der Winkel α₁ beträgt dann 0° und der Winkel β₁ von -1 bis -10° , bevorzugt von -2 bis -8 °, besonders bevorzugt von -3 bis -7° und ganz besonders bevorzugt von -4 bis -6°. Der Winkel α₁ kann jedoch auch entlang im Verlauf des Abschnittes 1 variieren.

Die Länge L₁ ergibt sich aus dem Durchmesser r₁ zu Anfang des Abschnittes 1 und dem Winkel β₁ durch einfach trigonometrische Berechnung.

Im Abschnitt 2 bleibt R₂ konstant und r₂ Null, α₂ und β₂ sind jeweils 0°.

Die zweite Konstruktionsalternative kann auch dadurch erreicht werden, daß man R₁ vergrößert und r₁ konstant hält. In diesem Fall beträgt der Winkel β₁ 0° und der Winkel α₁ von 0,0001 bis 10°, bevorzugt von 0,0002 bis 8 °, besonders bevorzugt von 0,0003 bis 7° und ganz besonders bevorzugt von 0,0003 bis 6 °.

Im Abschnitt 2 bleiben dann R₂ und r₂ konstant, α₂ und β₂ sind jeweils 0°.

In einer dritten Konstruktionsalternative bleibt die Fläche F₁ in einem Abschnitt 1 gleich und erweitert sich im zweiten Abschnitt um im dritten Abschnitt wieder konstant zu bleiben.

Dazu bleiben im Abschnitt 1 R₁ und r₁ konstant, α₁ und β₁ sind jeweils 0°.

Im zweiten Abschnitt vergrößert sich F₂, was durch ein anwachsende R₂ und ein konstantes r₂ erreicht wird. Dies bedeutet, daß β₂ gleich Null ist und α₂ beispielsweise von 0,0001 bis 10°, bevorzugt von 0,0002 bis 8 °, besonders bevorzugt von 0,0003 bis 7° und ganz besonders bevorzugt von 0,0003 bis 6 ° beträgt.

Im dritten Abschnitt bleibt F₃ konstant, dies wird erreicht durch R₃ und r₃ konstant, α₃ und β₃ sind jeweils 0°.

Diese dritte Konstruktionsalternative kann alternativ auch dadurch erreicht werden, daß im zweiten Abschnitt R₂ konstant gehalten wird und r₂ auf Null verringert wird. Der Winkel α₂ beträgt dann 0° und der Winkel β₂ von -1 bis -10° , bevorzugt von -2 bis -8 °, besonders bevorzugt von -3 bis -7° und ganz besonders bevorzugt von -4 bis -6°.

Die konstante Fläche in den Abschnitte 1 und 3 wird wiederum dadurch erreicht, daß man R₁ und r₁ konstant hält sowie R₃ und r₃ konstant hält.

In einer vierten Konstruktionsalternative wird in einem Abschnitt 1 die Fläche F₁ gleichgehalten, im Abschnitt 2 wird F₂ dann verringert und in Abschnitt 3 F₃ vergrößert und im Abschnitt 4 konstant gehalten wird.

Dies wird dadurch erreicht, daß man R₁ und r₁ konstant hält und α₁ und β₁ auf jeweils 0° hält.

Im zweiten Abschnitt verringert sich F₂, was durch ein verkleinertes R₂ und konstantes r₂ erreicht wird. Dazu wird β₂ auf 0° gehalten, α₂ beträgt beispielsweise weniger als 0° und mehr als -90°, bevorzugt -7 bis -65°, besonders bevorzugt -15 bis -35° und ganz besonders bevorzugt -20 bis -30°.

Im dritten Abschnitt vergrößert sich F₃, was erreicht wird durch ein anwachsende R₃ und ein konstantes r₃, was bedeutet, daß β₃ gleich Null ist und α₃ beispielsweise von 0,0001 bis 10°, bevorzugt von 0,0002 bis 8 °, besonders bevorzugt von 0,0003 bis 7° und ganz besonders bevorzugt von 0,0003 bis 6 °.

Im vierten Abschnitt bleibt F₄ dann konstant, dazu bleiben im Abschnitt 4 R₄ und r₄ konstant, α₄ und β₄ sind jeweils 0°.

Diese vierte Konstruktionsalternative kann alternativ auch folgendermaßen erreicht werden:
Man hält R₁ und r₁ konstant und hält α₁ und β₁ auf jeweils 0° (konstantes F₁).

Im zweiten Abschnitt verringert sich F₂, was durch ein verkleinertes R₂ und konstantes r₂ erreicht wird. Dazu wird β₂ auf 0° gehalten, α₂ beträgt beispielsweise weniger als 0° und mehr als -90°, bevorzugt -7 bis -65°, besonders bevorzugt -15 bis -35° und ganz besonders bevorzugt -20 bis -30°.

Dagegen wird im dritten Abschnitt die Flächenverringerung F₃ durch ein sich auf Null veringerndes r₃ und konstantes R₃ erreicht. Der Winkel α₃ beträgt dann 0° und der Winkel β₃ von -1 bis -10° , bevorzugt von -2 bis -8 °, besonders bevorzugt von -3 bis -7° und ganz besonders bevorzugt von -4 bis -6°.

Im vierten Abschnitt wird R₄ konstant gehalten und r₄=0. α₄ und β₄ sind jeweils 0°.

In einer fünften Konstruktionsalternative wird im ersten Abschnitt die Fläche F₁ verkleinert, im zweiten Abschnitt F₂ vergrößert und im dritten Abschnitt F₃ konstant gehalten. Im ersten Abschnitt verringert sich F₁, was durch ein sich verkleinerndes R₁ und konstantes r₁ erreicht wird. Dazu wird β₁ auf 0° gehalten, α₁ beträgt beispielsweise weniger als 0° und mehr als -90°, bevorzugt -7 bis -65°, besonders bevorzugt -15 bis -35° und ganz besonders bevorzugt -20 bis -30°.

Im zweiten Abschnitt vergrößert sich F₂, was erreicht wird durch ein anwachsendes R₂ und ein konstantes r₂, was bedeutet, daß β₂ gleich Null ist und α₂ beispielsweise von 0,0001 bis 10°, bevorzugt von 0,0002 bis 8 °, besonders bevorzugt von 0,0003 bis 7° und ganz besonders bevorzugt von 0,0003 bis 6 °.

Im Abschnitt 3 bleiben R₃ und r₃ konstant, α₃ und β₃ sind jeweils 0°.

In einer alternativen Ausführungsform der fünften Konstruktionsalternative kann man wie folgt verfahren:
Im ersten Abschnitt verringert sich F₁, was durch ein sich verkleinerndes R₁ und konstantes r₁ erreicht wird. Dazu wird β₁ auf 0° gehalten, α₁ beträgt beispielsweise weniger als 0° und mehr als -90°, bevorzugt -7 bis -65°, besonders bevorzugt -15 bis -35° und ganz besonders bevorzugt -20 bis -30°.

Die Flächenverringerung F₂ wird jetzt dadurch erreicht, daß im zweiten Abschnitt R₂ konstant gehalten wird und r₂ auf Null verringert wird. Der Winkel α₂ beträgt dann 0° und der Winkel β₂ von -1 bis -10° , bevorzugt von -2 bis -8 °, besonders bevorzugt von -3 bis -7° und ganz besonders bevorzugt von -4 bis -6°.

Im Abschnitt 3 bleiben R₃ und r₃ konstant, wobei R₃ = R₂ und r₃ = 0, α₃ und β₃ sind jeweils 0°.

In der sechsten Konstruktionsalternative wird die Fläche F₁ verringert, bleibt im Abschnitt zwei konstant, wird im Abschnitt 3 wieder vergrößert und bleibt im vierten Abschnitt dann wieder konstant.

Im ersten Abschnitt verringert sich F₁, was durch ein sich verkleinerndes R₁ und konstantes r₁ erreicht wird. Dazu wird β₁ auf 0° gehalten, α₁ beträgt beispielsweise weniger als 0° und mehr als -90°, bevorzugt -7 bis -65°, besonders bevorzugt -15 bis -35° und ganz besonders bevorzugt -20 bis -30°.

Im Abschnitt 2 bleiben R₂ und r₂ konstant, α₂ und β₂ sind jeweils 0°.

Im dritten Abschnitt vergrößert sich F₃, was erreicht wird durch ein anwachsendes R₃ und ein konstantes r₃, das auch gleich r₂ ist, was bedeutet, daß β₃ gleich Null ist und α₃ beispielsweise von 0,0001 bis 10°, bevorzugt von 0,0002 bis 8 °, besonders bevorzugt von 0,0003 bis 7° und ganz besonders bevorzugt von 0,0003 bis 6 °.

Im Abschnitt 4 bleiben R₄ und r₄ konstant, α₄ und β₄ sind jeweils 0°.

In einer alternativen Ausführungsform der sechsten Konstruktionsalternative kann man wie folgt verfahren:
Im ersten Abschnitt verringert sich F₁, was durch ein sich verkleinerndes R₁ und konstantes r₁ erreicht wird. Dazu wird β₁ auf 0° gehalten, α₁ beträgt beispielsweise weniger als 0° und mehr als -90°, bevorzugt -7 bis -65°, besonders bevorzugt -15 bis -35° und ganz besonders bevorzugt -20 bis -30°.

Im Abschnitt 2 bleiben R₂ und r₂ konstant, α₂ und β₂ sind jeweils 0°.

Die Flächenverringerung von F₃ wird jetzt dadurch erreicht, daß im dritten Abschnitt R₃ konstant gehalten wird und r₃ auf Null verringert wird. Der Winkel α₃ beträgt dann 0° und der Winkel β₃ von -1 bis -10° , bevorzugt von -2 bis -8 °, besonders bevorzugt von -3 bis -7° und ganz besonders bevorzugt von -4 bis -6°.

Im Abschnitt 4 bleiben R₄ und r₄ konstant, wobei R₄ = R₃ und r₄ = 0, α₄ und β₄ sind jeweils 0°.

In der sechsten Konstruktionsalternative wird die Länge des Abschnitts 2 je nach eingesetztem Amin unterschiedlich gewählt: Bei stark ablagerungsbildenden Isocyanaten, wie z.B. (cyclo)aliphatischen Isocyanaten und insbesondere 1,6-Hexamethylendiisocyanat, wird ein kurzer Abschnitt 2 bevorzugt.

Die Länge der Zone 2 L₂ ist in der Regel kleiner als das 30-fache der resultierenden Spaltbreite (R₂ - r₂), bevorzugt kleiner als das 15-fache, besonders bevorzugt kleiner als das 10-fache, ganz besonders bevorzugt kleiner als 6-fache, insbesondere kleiner als das fünffache und im speziellen kleiner als das dreifache. Oft ist L₂ kleiner als das zweifache und sogar kleiner als das einfache der Spaltbreite (R₂ - r₂).

Hingegen wird bei wenig ablagerungsbildenden Isocyanaten, wie beispielsweise aromatischen isocyanaten und insbesondere Toluylendiisocyanat, ein möglichst große Länge des Abschnitts 2 bevorzugt.

Die Länge der Zone 2 ist bei derartigen Isocyanaten größer als das einfache der resulierenden Spaltbreite (R₂-r₂), bevorzugt größer als das 5-fache, besonders bevorzugt größer als das 10-fache, ganz besonders bevorzugt größer als das 15-fache und im speziellen größer als 30-fache.

Die Länge des jeweils letzten Abschnitts mit gleichbeliebender Fläche F, also in der sechsten Konstruktionsalternative L₄, wird so gewählt, dass man einerseits die gewünschte mittlere Kontaktzeit im Reaktor erreicht und andererseits ein L₄/(2 * R₄)-Verhältnis von größer 2, bevorzugt größer 4, besonders bevorzugt größer 6, ganz besonders bevorzugt größer 10 und im speziellen größer 15 erreicht.

In Abschnitten gleicher oder zunehmender Fläche wird F so gewählt, dass die mittlere Geschwindigkeit des Reaktionsgemischs im allgemeinen größer ist als 60 m/s, bevorzugt größer als 100, besonders bevorzugt größer als 160 m/s und ganz besonders bevortzugt größer als 180 m/s ist und im speziellen größer 200 m/s. Zudem wird F so gewählt, dass die mittlere Geschwindigkeit im allgemeinen kleiner ist als 250 m/s, bevorzugt kleiner als 240, besonders bevorzugt kleiner als 230 m/s, ganz besonders bevorzugt kleiner als 220 m/s und im speziellen kleiner als 210 m/s ist.

Die Veränderung der Parameter R und r kann innerhalb der Abschnitte linear oder nicht-linear erfolgen. Bei einer linearen Änderung bleiben die Winkel α und β innerhalb der Abschnitte gleich und verändern sich erst bei den Übergängen zwischen den Abschnitten. Bei nicht-linearem Verlauf innerhalb der Abschnitte kann der Verlauf konkav, d.h. in den Strömungsraum geneigt, konvex, d.h. vom Strömungsraum wegweisend, oder gemischt konkav-konvex bzw. konvex-konkav sein. Ein konkaver Verlauf bedeutet für den Winkel α, daß dieser sich im Verlauf der Strömung vergrößert bzw. positiver wird, und für den Winkel β, daß sich dieser verkleinert, bzw. negativer wird. Bevorzugt wird ein linearer Verlauf.

Die Übergänge zwischen den jeweiligen Abschnitten 1 bis 4 können unabhängig voneinander stufig oder abgerundet ausgeführt werden. Bevorzugt wird eine abgerundete Ausführung.

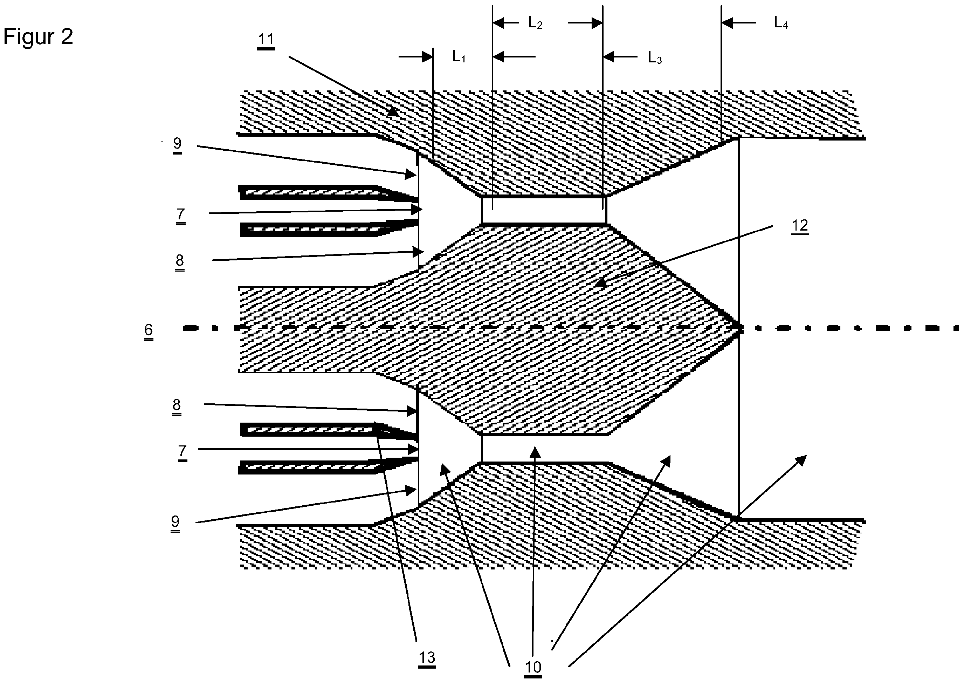

Die sechste Konstruktionsalternative ist in einer besonders bevorzugten Ausführungsform in Figur 2 (nicht maßstabsgerecht) wiedergegeben:
Um eine gedachte Achse 6 sind rotationssymmetrisch ein kreisförmiger Ringspalt 7 zur Eindosierung des Amins und ein innerer 8 sowie ein äußerer 9 kreisförmiger Ringspalt zur Eindosierung von Phosgen angeordnet. Der Reaktionsraum 10 ist durch eine äußere Reaktorwand 11 und einen inneren Volumenkörper 12 begrenzt und entlang dem Verlauf der Reaktion in vier Abschnitte der Länge L₁, L₂, L₃ und L₄ aufgeteilt, die sich durch ihre durchströmte Fläche unterscheiden. Im Verlauf des ersten Abschnitts, direkt nach der Vermischung der Eduktströme, wird die durchströmte Fläche F₁ durch Verkleinerung von R₁ und Vergrößerung von r₁ verringert, bleibt im Abschnitt 2 gleich, erweitert sich im Abschnitt 3 wieder durch Vergrößerung von R₃ und Verkleinerung von r₃ auf Null, um dann im vierten Abschnitt konstant zu bleiben.

Der Volumenkörper im Inneren des Rohrreaktors ist bevorzugt mit den Wänden des Reaktors zur Stabilisierung verbunden, bevorzugt über Streben oder Leitbleche. Die Form dieser Streben ist bevorzugt möglichst stromlinienförmig, beispielsweise mit einem Tropfenquerschnitt. Es ist aber auch möglich, diese Befestigungen zur zusätzlichen Erhöhung der Tangentialgeschwindigkeit des Reaktionsgemisches zu verwenden. so daß dies zu einer zusätzlichen Durchmischung führt.

Das durchströmte Volumen des Reaktors kann mit statischen Mischern ausgefüllt sein, beispielsweise Packungen, Formkörpern, Geweben, Loch- oder Schlitzblechen, bevorzugt ist das Volumen jedoch möglichst frei von Einbauten.

Denkbar ist auch der Einbau von Leitblechen in den Reaktionsraum. Ein geeignetes ein turbulenzerzeugendes Element wäre beispielsweise ein eingelassenes spiralförmig verdrilltes Band, runde oder eckige Schrägplatten oder dergleichen.

Nach der Reaktion wird das gasförmige Umsetzungsgemisch bevorzugt bei Temperaturen größer 130 °C mit einem Lösungsmittel gewaschen (Quench). Als Lösungsmittel sind bevorzugt Kohlenwasserstoffe, die gegebenenfalls mit Halogenatomen substituiert sind, geeignet, wie beispielsweise Hexan, Benzol, Nitrobenzol, Anisol, Chlorbenzol, Chlortoluol, o-Dichlorbenzol, Trichlorbenzol, Diethylisophthalat (DEIP), Tetrahydrofuran (THF), Dimethylformamid (DMF), Xylol, Chlornaphthalin, Decahydronaphthalin und Toluol. Als Lösungsmittel wird besonders bevorzugt Monochlorbenzol eingesetzt. Als Lösungsmittel kann auch das Isocyanat eingesetzt werden. Bei der Wäsche wird das Isocyanat selektiv in die Waschlösung übergeführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Nachdem das Reaktionsgemisch im Reaktionsraum umgesetzt wurde, führt man es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamylchlorids im gewählten Quenchmedium gehalten. Besonders bevorzugt wird dabei die Temperatur des inerten Lösungsmittels oberhalb der Schmelztemperatur des zum Amin gehörigen Carbamylchlorids gehalten

Im allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Die Wäsche kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.
Ein geeigneter Quench ist beispielsweise bekannt aus EP-A1 1403248, Sp. 2, Z. 39 - Sp. 3, Z. 18.

In dieser Quenchzone wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570°C auf 100 bis 200 °C, bevorzugt auf 140 bis 180 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das im Reaktionsgemisch enthaltene Isocyanat.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reaktionsraum entfernt und anschließend aufgearbeitet wird. Die verbleibende Gasphase wird über einen zweiten Auslass aus dem Reaktionsraum entfernt und ebenfalls aufgearbeitet.

Der Quench kann beispielsweise erfolgen, wie in der EP 1403248 A1 beschrieben, oder wie in der unveröffentlichten internationalen Anmeldung mit dem Aktenzeichen PCT/EP/05/006745 und dem Anmeldedatum 22.06.2005 beschrieben.

Die Flüssigkeitströpfchen werden dazu mittels Ein- oder Zweistoffzerstäuberdüsen, vorzugsweise Einstoffzerstäuberdüsen, erzeugt und erzeugen je nach Ausführungsform einen Sprühkegelwinkel von 10 bis 140°, bevorzugt von 10 bis 120°, besonders bevorzugt von 10° bis 100°.

Die Flüssigkeit, die über die Zerstäuberdüsen eingedüst wird, muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können.

In einer besonderen Ausführungsform des Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie HCl und Phosgen, aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung, ganz besonders bevorzugt wird in diesem Fall Monochlorbenzol verwendet.

Geringe Mengen von Nebenprodukten, die im Isocyanat verbleiben, können mittels zusätzlicher Rektifikation, durch Strippen mit einem Inertgas oder auch Kristallisation, bevorzugt durch Rektifikation vom erwünschten Isocyanat getrennt werden.

In der anschließenden optionalen Reinigungsstufe wird das Isocyanat, bevorzugt durch Destillation, vom Lösungsmittel abgetrennt. Ebenfalls kann hier noch die Abtrennung von restlichen Verunreinigungen, umfassend Chlorwasserstoff, Inertmedium und/oder Phosgen, erfolgen, wie beispielsweise beschrieben in DE-A1 10260092.

Ein weiterer Gegenstand der vorliegenden Erfindung ist eine Vorrichtung, umfassend mindestens einen Rohrreaktor, an dessen einer Stirnseite sich (2n + 1) ringförmig umeinander angeordnete Ringspalte befinden, wobei n eine positive ganze Zahl von mindestens 1 ist, bevorzugt ist n 1, 2 oder 3, besonders bevorzugt 1 oder 2 und ganz besonders bevorzugt ist n = 1, von denen jeder zweite Ringspalt mit mindestens einer Zuleitung für einen Eduktstrom und die dazwischenliegenden Ringspalte mit mindestens einer Zuleitung für einen anderen Eduktstrom verbunden sind, wobei die Ringspalte konzentrisch, besonders bevorzugt rotationssymmetrisch konzentrisch an der Stirnseite um die Längsachse des Reaktors angeordnet sind, und in deren Mitte sich ein in den Rohrreaktor hineinragender um die Längsachse des Reaktors angeordneter Volumenkörper befindet, wobei der Außendurchmesser des Volumenkörpers und der Innendurchmesser des Rohrreaktors einen Reaktionsraum einschließen.

Die zwischen Innenwand des Rohrreaktors und Außenwand des Volumenkörpers eingeschlossene Querschnittsfläche des Reaktionsraums kann bevorzugt so konstruiert werden, wie es in den oben angeführten Konstruktionsalternativen beschrieben ist.

Zusätzlich kann sich an der gegenüberliegenden Stirnseite des Rohrreaktors eine Verbindung zu einer weiteren Vorrichtung befinden, in der sich mindestens eine Zerstäuberdüse befindet, mit der eine Flüssigkeit mit dem den Rohrreaktor verlassenden Reaktionsgemisch in Kontakt gebracht werden kann.

In einer bevorzugten Ausführungsform der vorliegenden Erfindung kann die Vorrichtung so gestaltet sein, daß die Zuleitungen der Eduktströme, bevorzugt der Aminströme am Übergang zu den Ringspalten sich zu den Ringspalten hin verengen (13 in Figur 2) und dabei die Zuführungswandungen mit einem halben Öffungswinkel, also den Winkel zwischen Längsachse der Zuleitung und der sich verengenden Zuführungswandung, von mehr als 0° bis hin zu einschließlich 70° auslaufen, bevorzugt 1 bis 60°, besonders bevorzugt 2 bis 50°, besonders bevorzugt 3 bis 45°, ganz besonders bevorzugt 5 bis 40, insbesondere 7 bis 30 und speziell 7 bis 20°.

Die Länge der Verengung ergibt sich aus dem gewählten Winkel und dem Aussendurchmesser des Ringspalts und dem Innendurchmesser der zuführenden Rohrleitung.

Durch derartige Verengung und Winkel wird eine feststoffablagerungsarme Vermischung der unterschiedlichen Ströme bewirkt.

## Patentansprüche

1. Verfahren zur Herstellung von Isocyanaten durch Umsetzung der korrespondierenden Amine mit Phosgen, gegebenenfalls in Gegenwart von mindestens einem Inertmedium, in der Gasphase, in dem in einem Reaktor n Aminströme mit n+1 Phosgenströmen umgesetzt werden, wobei n eine positive ganze Zahl von mindestens 1 ist, und alle Amin- und Phosgenströme jeweils über Ringspalte zur Vermischung in den Reaktor dosiert werden, wobei die Ringspalte eine konzentrische Anordnung um die Längsachse des Reaktors aufweisen.

2. Verfahren nach Anspruch 1, **dadurch gekennzeichnet, daß** das Isocyanat ausgewählt ist aus der Gruppe bestehend aus 1,6-Diisocyanatohexan, 1-Isocyanato-3,3,5- trimethyl-5-(isocyanatomethyl)cyclohexan, 4,4'-Di(isocyanatocyclohexyhmethan und Toluylendiisocyanat-Isomerengemischen.

3. Verfahren nach einem der vorstehenden Ansprüche, **dadurch gekennzeichnet, daß** mindestens ein Inertmedium anwesend ist, ausgewählt aus der Gruppe bestehend aus Stickstoff, Helium, Argon, Chlorbenzol, Chlortoluol, o-Dichlorbenzol, Toluol, Xylol, Chlornaphthalin, Decahydronaphthalin, Kohlenstoffdioxid und Kohlenstoffmonoxid.

4. Verfahren gemäß Anspruch 3, **dadurch gekennzeichnet, daß** das Gasvolumen von Inertmedium zu Amin bzw. zu Phosgen mehr als 0,0001 bis 30 beträgt.

5. Verfahren nach einem der vorstehenden Ansprüche, **dadurch gekennzeichnet, daß** die Temperatur im Verfahren von 200 bis 600 °C beträgt.

6. Verfahren nach einem der vorstehenden Ansprüche, **dadurch gekennzeichnet, daß** man ein molares Verhältnis von Phosgen zu Aminogruppen von 1,1 :1 bis 20 : 1 einstellt.

7. Verfahren nach einem der vorstehenden Ansprüche, **dadurch gekennzeichnet, daß** man als äußersten Strom an der Außenwand und als innersten Strom jeweils einen Phosgenstrom in den Reaktor dosiert.

8. Verfahren nach einem der vorstehenden Ansprüche, **dadurch gekennzeichnet, daß** das Verhältnis der Flächen des jeweils außenliegenden Phosgenstroms zur Fläche des Aminstroms zur Fläche des jeweils benachbarten inneren Phosgenstroms von 0,3 - 5 : 1 : 0,3 bis 5 beträgt.

9. Verfahren nach einem der vorstehenden Ansprüche, **dadurch gekennzeichnet, daß** das Verhältnis der Gesamtfläche der Aminströme zur Gesamtfläche der Phosgenströme größer als 0,00002 und kleiner als 5 ist.

10. Verfahren nach einem der vorstehenden Ansprüche, **dadurch gekennzeichnet, daß** man die einzelnen Edukte in der Mischeinrichtung mit einer Strömungsgeschwindigkeit von 20 bis 400 Meter/Sekunde in den Reaktor führt.

11. Verfahren nach einem der vorstehenden Ansprüche, **dadurch gekennzeichnet, daß** es sich bei dem Reaktor, in dem man die Reaktion durchführt, um einen Ringspaltraum handelt.

12. Verfahren nach Anspruch 10, **dadurch gekennzeichnet, daß** der Reaktor zur Ausformung des Ringspaltraumes einen Volumenkörper im Inneren enthält.

13. Verfahren nach Anspruch 11, **dadurch gekennzeichnet, daß** man den Volumenkörper im Verlauf des Reaktors auf einen Durchmesser von Null verjüngt.

14. Verfahren nach Anspruch 10, **dadurch gekennzeichnet, daß** sich die durchströmte Fläche des Reaktors während des Durchgangs durch den Reaktor nicht ändert.

15. Verfahren nach Anspruch 10, **dadurch gekennzeichnet, daß** sich die durchströmte Fläche des Reaktors während des Durchgangs durch den Reaktor in einem ersten Abschnitt vergrößert und anschließend in einem zweiten Abschnitt konstant bleibt.

16. Verfahren nach Anspruch 10, **dadurch gekennzeichnet, daß** die durchströmte Fläche des Reaktors während des Durchgangs durch den Reaktor in einem ersten Abschnitt konstant bleibt, sich in einem zweiten Abschnitt erweitert und in einem dritten Abschnitt konstant bleibt.

17. Verfahren nach Anspruch 10, **dadurch gekennzeichnet, daß** die durchströmte Fläche des Reaktors während des Durchgangs durch den Reaktor in einem ersten Abschnitt konstant bleibt, sich in einem zweiten Abschnitt veringert, in einem dritten Abschnitt erweitert und in einem vierten Abschnitt konstant bleibt.

18. Verfahren nach Anspruch 10, **dadurch gekennzeichnet, daß** sich die durchströmte Fläche des Reaktors während des Durchgangs durch den Reaktor in einem ersten Abschnitt verkleinert, in einem zweiten Abschnitt vergrößert und in einem dritten Abschnitt konstant bleibt.

19. Verfahren nach Anspruch 10, **dadurch gekennzeichnet, daß** sich die durchströmte Fläche des Reaktors während des Durchgangs durch den Reaktor in einem ersten Abschnitt verkleinert, in einem zweiten Abschnitt konstant bleibt, sich im dritten Abschnitt vergrößert und in einem vierten Abschnitt konstant bleibt.

20. Verfahren nach einem der vorstehenden Ansprüche, **dadurch gekennzeichnet, daß** die mittlere Kontaktzeit des Umsetzungsgemisches zwischen 0,001 Sekunden und weniger als 5 Sekunden beträgt.

21. Verfahren nach einem der vorstehenden Ansprüche, **dadurch gekennzeichnet, daß** die Strömung im Reaktor eine Bodenstein-Zahl von mehr als 10 aufweist.

22. Verfahren nach einem der vorstehenden Ansprüche, **dadurch gekennzeichnet, daß** die Strömung des Reaktionsgemischs eine Reynolds-Zahl von mindestens 2300 aufweist.

23. Verfahren nach einem der vorstehenden Ansprüche, **dadurch gekennzeichnet, daß** der Umsatz an Amin in dem Raum, in dem die Vermischung der Edukte zu einem Grad von 99% stattfindet, nicht mehr als 15% beträgt.

24. Vorrichtung, umfassend mindestens einen Rohrreaktor, an dessen einer Stirnseite sich (2n + 1) ringförmig umeinander angeordnete Ringspalte befinden, wobei n eine positive ganze Zahl von mindestens 1 ist, von denen jeder zweite Ringspalt mit mindestens einer Zuleitung für einen Eduktstrom und die dazwischenliegenden Ringspalte mit mindestens einer Zuleitung für einen anderen Eduktstrom verbunden sind, wobei die Ringspalte konzentrisch an der Stirnseite um die Längsachse des Reaktors angeordnet sind, und in deren Mitte sich ein in den Rohrreaktor hineinragender um die Längsachse des Reaktors angeordneter Volumenkörper befindet, wobei der Außendurchmesser des Volumenkörpers und der Innendurchmesser des Rohrreaktors einen Reaktionsraum einschließen.

25. Verwendung einer Vorrichtung gemäß Anspruch 24 in einer Gasphasenphosgenierung.

## Claims

1. A process for preparing isocyanates by reacting the corresponding amines with phosgene, optionally in the presence of at least one inert medium, in the gas phase, in which process n amine streams are reacted with n+1 phosgene streams in a reactor, where n is a positive integer of at least 1, and all amine and phosgene streams are introduced into the reactor via annular gaps for mixing, with the annular gaps having a concentric arrangement around the longitudinal axis of the reactor.

2. The process according to claim 1, wherein the isocyanate is selected from the group consisting of 1,6-diisocyanatohexane, 1-isocyanato-3,3,5-trimethyl-5-(isocyanatomethyl)cyclohexane, 4,4'-di(isocyanatocyclohexyl)methane and tolylene diisocyanate isomer mixtures.

3. The process according to either of the preceding claims, wherein at least one inert medium selected from the group consisting of nitrogen, helium, argon, chlorobenzene, chlorotoluene, o-dichlorobenzene, toluene, xylene, chloronaphthalene, decahydronaphthalene, carbon dioxide and carbon monoxide is present.

4. The process according to claim 3, wherein the gas volume of inert medium to amine or to phosgene is from > 0.0001 to 30.

5. The process according to any of the preceding claims, wherein the temperature in the process is from 200 to 600°C.

6. The process according to any of the preceding claims, wherein a molar ratio of phosgene to amino groups of from 1.1:1 to 20:1 is set.

7. The process according to any of the preceding claims, wherein the outermost stream introduced into the reactor at the outer wall and the innermost stream introduced into the reactor are each a phosgene stream.

8. The process according to any of the preceding claims, wherein the ratio of the areas of the outer phosgene stream in question to the area of the amine stream to the area of the adjacent inner phosgene stream in question is 0.3-5:1:0.3-5.

9. The process according to any of the preceding claims, wherein the ratio of the total area of the amine streams to the total area of the phosgene streams is greater than 0.00002 and less than 5.

10. The process according to any of the preceding claims, wherein the individual starting materials are conveyed in the mixing device at a flow velocity of from 20 to 400 meters/second into the reactor.

11. The process according to any of the preceding claims, wherein the reactor in which the reaction is carried out is an annular gap space.

12. The process according to claim 10, wherein the reactor comprises a volume body in its interior to form the annular gap space.

13. The process according to claim 11, wherein the volume body tapers to a diameter of zero along the reactor.

14. The process according to claim 10, wherein the area of the reactor through which flow occurs does not change during passage through the reactor.

15. The process according to claim 10, wherein the area of the reactor through which flow occurs increases in a first section during passage through the reactor and subsequently remains constant in a second section.

16. The process according to claim 10, wherein the area of the reactor through which flow occurs remains constant in a first section during passage through the reactor, increases in a second section and remains constant in a third section.

17. The process according to claim 10, wherein the area of the reactor through which flow occurs remains constant in a first section during passage through the reactor, decreases in a second section, increases in a third section and remains constant in a fourth section.

18. The process according to claim 10, wherein the area of the reactor through which flow occurs decreases in a first section during passage through the reactor, increases in a second section and remains constant in a third section.

19. The process according to claim 10, wherein the area of the reactor through which flow occurs decreases in a first section during passage through the reactor, remains constant in a second section, increases in the third section and remains constant in a fourth section.

20. The process according to any of the preceding claims, wherein the mean contact time of the reaction mixture is between 0.001 seconds and less than 5 seconds.

21. The process according to any of the preceding claims, wherein the flow in the reactor has a Bodenstein number of more than 10.

22. The process according to any of the preceding claims, wherein the flow of the reaction mixture has a Reynolds number of at least 2300.

23. The process according to any of the preceding claims, wherein the conversion of amine in the space in which the mixing of the starting materials takes place to a degree of 99% is not more than 15%.

24. An apparatus comprising at least one tube reactor whose one end face is provided with (2n + 1) annular gaps arranged in a circular fashion around one another, where n is a positive integer of at least 1, of which each second annular gap is connected to at least one feed line for a feed stream and the annular gaps located in between are connected to at least one feed line for another feed stream, with the annular gaps being arranged concentrically around the longitudinal axis of the reactor at the end face and a volume body which is arranged around the longitudinal axis of the reactor and projects into the tube reactor being located in the middle thereof, with the external diameter of the volume body and the internal diameter of the tube reactor enclosing a reaction space.

25. The use of an apparatus according to claim 24 in a gas-phase phosgenation.

## Revendications

1. Procédé pour la préparation d'isocyanates par transformation des amines correspondantes avec du phosgène, le cas échéant en présence d'au moins un milieu inerte, dans la phase gazeuse, dans lequel, dans un réacteur, n flux d'amine sont transformés avec n+1 flux de phosgène, n étant un nombre entier positif d'au moins 1 et tous les flux d'amine et de phosgène étant à chaque fois dosés via des fentes annulaires pour le mélange dans le réacteur, les fentes annulaires présentant une disposition concentrique autour de l'axe longitudinal du réacteur.

2. Procédé selon la revendication 1, **caractérisé en ce que** l'isocyanate est choisi dans le groupe constitué par le 1,6-diisocyanatohexane, le 1-isocyanato-3,3,5-triméthyl-5-(isocyanatométhyl)cyclohexane, le 4,4'-di(isocyanatocyclohexyl)méthane et les mélanges d'isomères de toluylènediisocyanate.

3. Procédé selon l'une quelconque des revendications précédentes, **caractérisé en ce qu'**au moins un milieu inerte est présent, choisi dans le groupe constitué par l'azote, l'hélium, l'argon, le chlorobenzène, le chlorotoluène, l'o-dichlorobenzène, le toluène, le xylène, le chloronaphtalène, le décahydronaphtalène, le dioxyde de carbone et le monoxyde de carbone.

4. Procédé selon la revendication 3, **caractérisé en ce que** le volume gazeux du milieu inerte à celui de l'amine ou, selon le cas, du phosgène est supérieur à 0,0001 jusqu'à 30.

5. Procédé selon l'une quelconque des revendications précédentes, **caractérisé en ce que** la température dans le procédé est de 200 à 600°C.

6. Procédé selon l'une quelconque des revendications précédentes, **caractérisé en ce qu'**on règle un rapport molaire du phosgène aux groupes amino de 1,1:1 à 20:1.

7. Procédé selon l'une quelconque des revendications précédentes, **caractérisé en ce qu'**on dose, comme flux le plus externe au niveau de la paroi externe et comme flux le plus interne, à chaque fois un flux de phosgène dans le réacteur.

8. Procédé selon l'une quelconque des revendications précédentes, **caractérisé en ce que** le rapport des surfaces du flux de phosgène à chaque fois externe à la surface du flux d'amine à la surface du flux de phosgène interne à chaque fois adjacent est de 0,3-5:1:0,3 à 5.

9. Procédé selon l'une quelconque des revendications précédentes, **caractérisé en ce que** le rapport de la surface totale des flux d'amine à la surface totale des flux de phosgène est supérieur à 0,00002 et inférieur à 5.

10. Procédé selon l'une quelconque des revendications précédentes, **caractérisé en ce qu'**on guide les différents produits de départ dans le dispositif de mélange à une vitesse d'écoulement de 20 à 400 m/s dans le réacteur.

11. Procédé selon l'une quelconque des revendications précédentes, **caractérisé en ce qu'**il s'agit, pour le réacteur dans lequel on effectue la réaction, d'une chambre à fente annulaire.

12. Procédé selon la revendication 10, **caractérisé en ce que**, pour la réalisation de la chambre à fente annulaire, le réacteur contient un corps volumique en son sein.

13. Procédé selon la revendication 11, **caractérisé en ce qu'**on rétrécit le corps volumique à un diamètre de zéro le long du réacteur.

14. Procédé selon la revendication 10, **caractérisé en ce que** la surface traversée du réacteur, pendant le passage à travers le réacteur, ne se modifie pas.

15. Procédé selon la revendication 10, **caractérisé en ce que** la surface traversée du réacteur, pendant le passage à travers le réacteur, augmente dans une première section et reste ensuite constante dans une deuxième section.

16. Procédé selon la revendication 10, **caractérisé en ce que** la surface traversée du réacteur, pendant le passage à travers le réacteur, reste constante dans une première section, augmente dans une deuxième section et reste constante dans une troisième section.

17. Procédé selon la revendication 10, **caractérisé en ce que** la surface traversée du réacteur, pendant le passage à travers le réacteur, reste constante dans une première section, diminue dans une deuxième section, augmente dans une troisième section et reste constante dans une quatrième section.

18. Procédé selon la revendication 10, **caractérisé en ce que** la surface traversée du réacteur, pendant le passage à travers le réacteur, diminue dans une première section, augmente dans une deuxième section et reste constante dans une troisième section.

19. Procédé selon la revendication 10, **caractérisé en ce que** la surface traversée du réacteur, pendant le passage à travers le réacteur, diminue dans une première section, reste constante dans une deuxième section, augmente dans la troisième section et reste constante dans une quatrième section.

20. Procédé selon l'une quelconque des revendications précédentes, **caractérisé en ce que** le temps de contact moyen du mélange de transformation vaut entre 0,001 seconde et moins de 5 secondes.

21. Procédé selon l'une quelconque des revendications précédentes, **caractérisé en ce que** l'écoulement dans le réacteur présente un nombre de Bodenstein supérieur à 10.

22. Procédé selon l'une quelconque des revendications précédentes, **caractérisé en ce que** l'écoulement du mélange réactionnel présente un nombre de Reynolds d'au moins 2300.

23. Procédé selon l'une quelconque des revendications précédentes, **caractérisé en ce que** la conversion de l'amine, dans la chambre dans laquelle a lieu le mélange des produits de départ à un degré de 99%, n'est pas supérieure à 15%.

24. Dispositif, comprenant au moins un réacteur tubulaire, présentant au niveau de sa face avant (2n + 1) fentes annulaires disposées de manière annulaire les unes autour des autres, n étant un nombre entier positif d'au moins 1, dont chaque deuxième fente annulaire est reliée à au moins une conduite d'alimentation pour un flux de produit de départ et les fentes annulaires se trouvant entre celles-ci sont reliées à au moins une conduite d'alimentation pour un autre flux de produit de départ, les fentes annulaires étant disposées de manière concentrique, au niveau de la face avant, autour de l'axe longitudinal du réacteur et un corps volumique pénétrant dans le réacteur tubulaire, disposé autour de l'axe longitudinal du réacteur, se trouvant en leur centre, le diamètre externe du corps volumique et le diamètre interne du réacteur tubulaire renfermant une chambre de réaction.

25. Utilisation d'un dispositif selon la revendication 24 dans une phosgénation en phase gazeuse.
